# EUROPEAN PATENT SPECIFICATION

(11) **EP 3 103 356 B1**
(45) Date of publication and mention of the grant of the patent: **13.05.2020**
(21) Application number: 15878448.8
(22) Date of filing: 30.03.2015
(51) Int. Cl.: A24F 47/00, A61M 15/06, A61M 11/04

(54) **ATOMIZER AND ELECTRONIC CIGARETTE THEREOF**
ZERSTÄUBER UND ELEKTRONISCHE ZIGARETTE DAMIT
ATOMISEUR ET CIGARETTE ÉLECTRONIQUE ASSOCIÉE

(30) Priority: 20.01.2015 CN 201510027925
(43) Date of publication of application: 14.12.2016
(73) Proprietor: Joyetech (Changzhou) Electronics Co., Ltd., Changzhou Jiangsu JS 213125 (CN)
(72) Inventor: WEIHUA, Qiu, Changzhou Jiangsu 213125 (CN)
(74) Representative: CMS Cameron McKenna Nabarro Olswang LLP
(86) International application number: PCT/CN2015/075361
(87) International publication number: WO 2016/115771

(56) References cited:
- EP-A1- 2 823 720
- WO-A2-2014/195859
- CN-A- 1 541 577
- CN-A- 103 462 224
- CN-A- 103 750 570
- CN-A- 104 082 863
- CN-U- 202 014 571
- CN-U- 204 180 939
- US-A1- 2014 314 397

## Description

### FIELD

The present disclosure relates to an atomizer and an electronic cigarette with same, and more particularly, to an atomizer with side liquid injection and an electronic cigarette with same.

### BACKGROUND

The electronic cigarette, also known as virtual cigarette, electronic atomizer, and electronic cigar, has similar taste as that of an ordinary cigarette.

An atomizer is a part of the electronic cigarette. There are mainly two ways of liquid injection for the atomizer currently on the market: top liquid injection and basal liquid injection. Wherein, by the top liquid injection, it is usually required to open the top cover of the atomizer or remove the atomizer from the electronic cigarette so as to expose the injection port for injecting liquid. Due to the communication between the liquid storage chamber and the atomizing chamber and the specified hydraulic pressure difference therebetween, the pressure generated during the top liquid injection tends to cause the cigarette liquid pass through the cigarette liquid chamber and the liquid inlet and flow into the atomizing chamber, resulting in the atomizer leakage. Additionally, the operation of the basal liquid injection is troublesome, which requires to invert the electronic cigarette and open the base for liquid injection. Since the base is connected to the atomizer by screw thread, cigarette liquid are likely to spill from the thread connection part. Both the two ways may lead to complicated operation of the liquid injection of the atomizer, and the difficulty to clean the atomizer and/or the electronic cigarette

Documents EP 2 823 720, CN 104 082 863 and WO 2014/195859 also disclose electronic cigarettes with injection ports.

### SUMMARY

In view of the above, it is necessary to provide an atomizer with a side liquid injection to overcome the above defects.

The invetion is defined in the appended claims. Described herein is an atomizer, including: an atomizing pipe on which an injection port is provided; a liquid storage chamber provided within the atomizing pipe; and a liquid retainer movably arranged on the atomizing pipe, the liquid retainer being moveable vertically or laterally, or in a circle around the atomizing pipe, or rotatable around a point, wherein the liquid retainer is configured to adjust a relative state of the injection port with respect to the external environment, wherein the relative state includes that the injection port is in communication with the external environment or the injection port is not in communication with the external environment.

Preferably, the liquid retainer is provided with an injection window capable of being in communication with the injection port.

Preferably, the liquid retainer is a liquid retaining ring arranged on the atomizing pipe.

Preferably, the atomizing pipe defines a guiding groove for the liquid retainer to move vertically or laterally, and the liquid retainer is slidably mounted in the guiding groove.

Preferably, wherein the liquid retainer has one end coupled to an elastic element along an axial direction; the injection port of the atomizing pipe and the injection window on the liquid retainer are superposed when the liquid retainer is moved towards the end provided by the elastic element, allowing the injection of liquid.

Preferably, the liquid retainer is a liquid retaining block mounted on the atomizing pipe.

Preferably, one end of the liquid retainer is rotatablely mounted on the atomizing pipe by being fixed on a rotating axis, and the other end of the liquid retainer is releasable locked on the atomizing pipe.

Preferably, the atomizer further includes at least one stopper configured to removably clamp the liquid retainer on the atomizing pipe.

Preferably, the stopper is coupled to an inner wall of the liquid retainer, an outer wall of the atomizing pipe defining a limiting groove configured to couple with the stopper.

Preferably, the atomizer further includes two sealing elements located between the liquid retainer and the atomizing pipe, the injection port is located between the two sealing elements.

Preferably, the two sealing elements seal the atomizer to prevent cigarette liquid in the liquid storage chamber from spilling from the liquid storage chamber.

Preferably, the atomizing pipe defines two sealing grooves at two opposite sides of the injection port, the two sealing elements are received in the two sealing grooves respectively.

The present disclosure also provides an electronic cigarette including the above atomizer.

The atomizer and the electronic cigarette including the atomizer can change the relative state of the injection port with respect to the external environment during the movements of the liquid retainer, to achieve the side liquid injection of the atomizer. In this way, the atomizer and the electronic cigarette can meet the user's requirements of simple operation, easy injection and avoiding liquid leakage.

### BRIEF DESCRIPTION OF THE DRAWINGS

FIG. 1 is a diagrammatic view of an atomizer of the present disclosure.
FIG. 2 is a sectional view of the atomizer of the present disclosure.
FIG. 3 is a diagrammatic view of an adjusting structure of the atomizer of the present disclosure.
FIG. 4 is a diagrammatic view of an electronic cigarette of the present disclosure.

| | | |
|---|---|---|
| atomizer 100 | top cover 120 | liquid retainer 130 |
| injection window 131 | stopper 132 | sealing element 150 |
| liquid storage chamber 160 | atomizing pipe 170 | injection port 171 |
| sealing groove 172 | first pipe 173 | second pipe 175 |
| limiting groove 177 | atomizing unit 180 | base 190 |
| gas outlet pipe 200 | atomizing chamber 210 | electronic cigarette 300 |
| cigarette holder 310 | battery pack 320 | |

### DETAILED DESCRIPTION

In order to easily understand purpose, character and advantage of the present disclosure, the following is combined the drawings to describe specific implementation ways of the present disclosure in detail. The following description describes much specific to be convenient to fully understand the present disclosure. But the present disclosure can be implemented by other ways different from the following description, the person in the art can make similar improvement without violation of the spirit of the present disclosure, therefore the present disclosure is not restricted by the following concrete embodiments.

It should be explained that, when a feature or element is herein referred to as being "on" another feature or element, it can be directly on the other feature or element or intervening features and/or elements may also be present. It will also be understood that, when a feature or element is referred to as being "connected", "attached" or "coupled" to another feature or element, it can be directly connected, attached or coupled to the other feature or element or intervening features or elements may be present.

Unless otherwise defined, all the technical and scientific terms in the present disclosure have the meaning same to that understood by the technical person in the art. The terms in the specification of the present disclosure are only for describing the specific embodiments, are not intended to limit the present disclosure. The use of the term "and/or" in the description includes one or more related affairs purpose by any and all combinations.

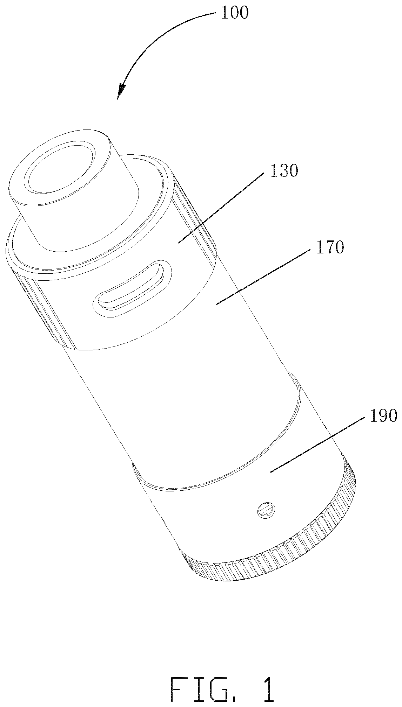

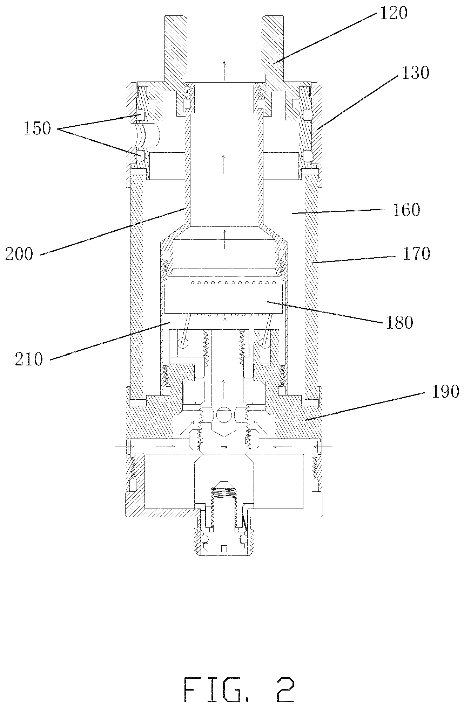

Please refer to FIGS. 1-2. A preferred embodiment of the present disclosure provides an atomizer 100. The atomizer 100 includes a top cover 120, a liquid retainer 130, a liquid storage chamber 160, an atomizing pipe 170, an atomizing assembly 180 and a base 190. The opposite ends of the atomizing pipe 170 are coupled to the top cover 120 and the base 190 respectively. The liquid storage chamber 160 and the atomizing assembly 180 are positioned within the atomizing pipe 170. The liquid retainer 130 is movably arranged on the atomizing pipe 170. The atomizing assembly 180 is fixed on one end of the base 190. In the embodiment, the top cover 120 or the base 190 may limit the liquid retainer 130 to move along an axial direction of the atomizing pipe 170. In other embodiments, the movements of the liquid retainer 130 can be limited by other ways, as long as it is convenient for injection and avoiding liquid leakage.

The atomizing pipe 170 is provided with an injection port 171 configured to make the inside of the atomizing pipe 170 in communication with the external environment, that is, the user may use the injection port 171 to move liquid into the liquid storage chamber 160.

The liquid retainer 130 is provided with an injection window 131 capable of being in communication with the injection port 171. The size of the injection port 171 may be larger, smaller or equal to the size of the injection window 131. In the embodiment, the liquid retainer 130 may be a liquid retaining ring rotatably arranged on the atomizing pipe 170. The injection port 171 and the injection window 131 may be superposed, and the injection port 171 may be in communication with the external environment, by turning the liquid retainer 130 (which may drive the injection window 131 provided thereon to rotate), to achieve the side injection of the atomizing pipe 170. Specifically, when turning the liquid retainer 130, if the injection port 171 is covered by the liquid retainer 130, the atomizer 100 is in a closed state, and if the injection window 131 and the injection port 171 are superposed, the atomizer 100 is in a state to be injected and is injectable.

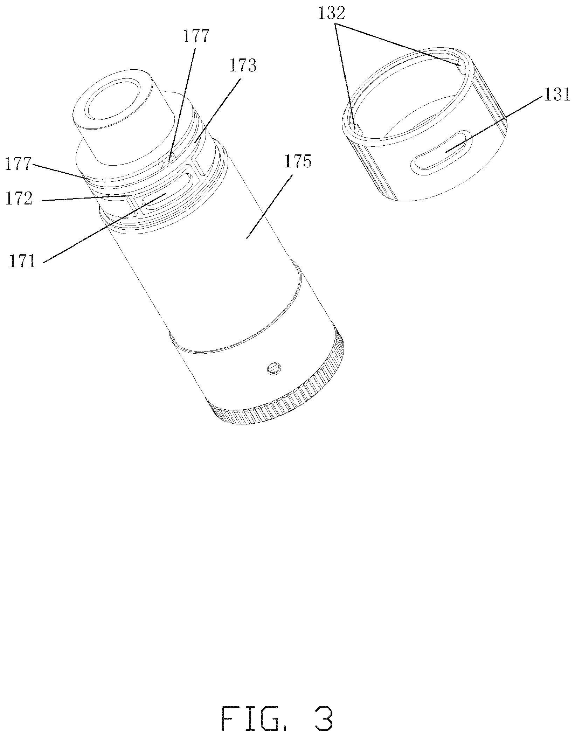

Also see FIG. 3, further, a sealing groove 172 configured to mount a sealing element 150 is arranged on an outer diameter surface of the atomizing pipe 170 and around the injection port 171. The sealing element 150 is arranged between the atomizing pipe 170 and the liquid retainer 130. The sealing element 150 is configured to seal the cigarette liquid to prevent the cigarette liquid from spilling from the liquid storage chamber 160 when the atomizer 100 is in a closed state. The sealing element 150 may be made of silicone, rubber or others. In the embodiment, there are two sealing grooves 172, and each sealing groove 172 is mounted with a sealing element 150.

Further, the atomizer 100 includes a stopper 132 configured to removably hold the liquid retainer 130 on the atomizing pipe 170, to prevent the user from turning the liquid retainer 130 to a state to be injected which may cause the cigarette liquid in the liquid storage chamber 160 spill out. In the embodiment, the stopper 132 is arranged on the inner wall of the liquid retainer 130. The outer wall of the atomizing pipe 170 is provided with a limiting groove 177 that matches the stopper 132. The stopper 132 may be an elastic element. When the user turn the liquid retainer 130 to drive the stopper 132 to slide into the limiting groove 177, the liquid retainer 130 may be clamped to prevent the user from turning the liquid retainer 130 to a state to be injected. It should be understood that, in other embodiments, when the stopper 132 is held in the limiting groove 177, the atomizer 100 is in a state to be injected, and when the stopper 132 is removed from the limiting groove 177, the atomizer 100 is in a closed state.

It should be understood that, the stopper 132 may be arranged on the atomizer 100, and the limiting groove 177 may be arranged on the liquid retainer 130. However, the implementation is not limited to the embodiment.

Further, the atomizing pipe 170 includes a first pipe 173 and a second pipe 175 which matches the first pipe 173. The injection port 171 and the limiting groove 177 are arranged on the first pipe 173 respectively. The first pipe 173 may be made of metal material, and the second pipe 175 may be made of translucent material, such as plastics or glass.

It should be understood that the first pipe 173 and the second pipe 175 may be made of the same material, and may be also made of different materials and not limited to the embodiment.

It should be understood that the atomizing pipe 170 is integrally molded, or formed of two components removably or non-removably connected.

Further, the atomizer 100 includes a gas outlet pipe 200. One end of the gas outlet pipe 200 is fixed on the top cover 120, and the other end of the gas outlet pipe 200 is fixed on the base 190. An atomizing chamber 210 is arranged within the gas outlet pipe 200. The atomizing assembly 180 is mounted within the atomizing chamber 210. When the cigarette liquid in the liquid storage chamber 160 flows into the atomizing chamber 210 through a liquid access port in the gas outlet pipe 200, the cigarette liquid may be absorbed by delivery cotton of the atomizing assembly 180, and be atomized by a heating element of the atomizing assembly 180.

During the liquid injection of the atomizer 100 according to the present invention, none of the following operations are required: for example, opening the top cover 120, disassembling the atomizer 100 from the electronic cigarette, or opening the base 190. The liquid injection of the atomizer 100 according to the present disclosure is simple and convenient. Further, there is no pressure generated upon the cigarette liquid in the liquid storage chamber 160 to cause the cigarette liquid flow towards the atomizing chamber 210, and there is no cigarette liquid spilling from the injection port. Thus, the present disclosure has effectively solved the problems in the prior art: the top liquid injection may easily generate a pressure upon the cigarette liquid in the liquid storage chamber 160 by the cigarette liquid flow towards the atomizing chamber 210, which causes leakage of the atomizer 100, whilst the basal liquid injection is troublesome in operation, easy to cause cigarette liquid spilling out and hard to clean the remained cigarette liquid in the atomizer.

In another embodiment, one end of the liquid retainer 130 is rotatably mounted on the atomizing pipe 170 by being fixed on a rotating shaft, and the other end of the liquid retainer 130 is releasable locked on the atomizing pipe 170. When the other end of the liquid retainer 130 is released from the atomizing pipe 170, the injection port 171 may be in communication with the external environment by rotating the liquid retainer 130, such that the liquid injection is possible.

In another embodiment, the liquid retainer 130 may be a liquid retaining ring arranged on the atomizing pipe 170 and is vertically moveable. One end of the liquid retainer 130 is provided with an elastic element; when the liquid retainer 130 moves towards the end provided with the elastic element, the cigarette liquid is injectable once the injection port 171 of the atomizing pipe 170 and the injection window 131 on the liquid retainer 130 are superposed. At the end of the liquid injection, by a reverse operation, the liquid retainer 130 can return to its initial position, and the atomizer 100 is in closed state.

In another embodiment, the top and bottom sides or the left and right sides of the injection port 171 of the atomizing pipe 170 are provided with guiding grooves. The liquid retainer 130 is movably fitted in the guiding groove to realize the vertical or lateral movements. If the user moves the liquid retainer 130 to communicate the atomizing pipe 170 with the external environment, the atomizer 100 is in a state to be injected; otherwise the atomizer 100 is in a closed state.

It should be understood that, the liquid retainer 130 is not limited to a liquid retaining ring in other embodiments, and the liquid retainer 130 can be a liquid retaining block of other shapes, such as in a shape of half ring, strip or sector.

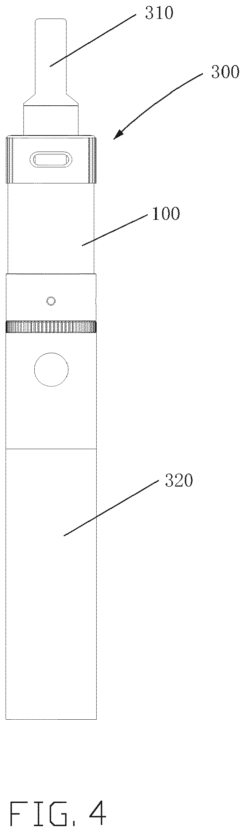

Please refer to FIG. 4. The present disclosure also provides an electronic cigarette 300 including the above atomizer 100. The electronic cigarette 300 further includes a cigarette holder 310 inserted into the top cover 120, and a battery pack 320 electrically connected with the atomizer 100.

The above are preferred embodiments of the disclosure described in detail, and should not be deemed as limitations to the scope of the present invention. It should be noted that variations and improvements will become apparent to those skilled in the art to which the present disclosure pertains without departing from its scope. Therefore, the scope of the present disclosure is defined by the appended claims.

## Claims

1. An atomizer (100), comprising an atomizing pipe (170) and a liquid storage chamber (160), the atomizer (100) being **characterized by**:
the atomizing pipe (170) defining an injection port (171);
the liquid storage chamber (160) being positioned in the atomizing pipe (170); and
the atomizer (100) further comprising a liquid retainer (130), the liquid retainer (130) defining an injection window (131) capable of being in communication with the injection port(171), the liquid retainer (130) being movably arranged on the atomizing pipe (170), the liquid retainer (130) being moveable vertically or laterally, or moveable in a circle around the atomizing pipe (170), or rotatable around a point, and the liquid retainer (130) being configured to adjust a relative state of the injection port (171) with respect to external environment, wherein the relative state is the injection port (171) being in communication with the external environment or the injection port (171) being not in communication with the external environment.

2. The atomizer (100) of claim 1, wherein the liquid retainer (130) is a liquid retaining ring arranged on the atomizing pipe (170).

3. The atomizer (100) of claim 2, wherein the atomizing pipe (170) defines a guiding groove for the liquid retainer (130) to move vertically or laterally, and the liquid retainer (130) is slidably mounted in the guiding groove.

4. The atomizer (100) of claim 2, wherein the liquid retainer (130) has one end provided with an elastic element; when the liquid retainer (130) is moved towards the end provided by the elastic element, and the injection port (171) of the atomizing pipe (170) and the injection window (131) on the liquid retainer (130) are superposed, the injection of cigarette liquid is allowed.

5. The atomizer (100) of claim 1, wherein the liquid retainer (130) is a liquid retaining block mounted on the atomizing pipe (170).

6. The atomizer (100) of claim 5, wherein one end of the liquid retainer (130) is rotatably mounted on the atomizing pipe (170) by fixing of a rotating shaft, and the other end of the liquid retainer (130) is releasably locked on the atomizing pipe (170).

7. The atomizer (100) of claim 5, wherein the atomizing pipe (170) defines a guiding groove for the liquid retainer (130) to move vertically or laterally, and the liquid retainer (130) is slidably mounted in the guiding groove.

8. The atomizer (100) of claim 1, wherein the atomizer (100) further comprises at least one stopper (132) configured to releasably clamp the liquid retainer (130) on the atomizing pipe (170).

9. The atomizer (100) of claim 8, wherein the stopper (132) is coupled to an inner wall of the liquid retainer (130), an outer wall of the atomizing pipe (170) defining a limiting groove (177) configured to couple with the stopper (132).

10. The atomizer (100) of claim 1, further comprising two sealing elements (150) located between the liquid retainer (130) and the atomizing pipe (170).

11. The atomizer (100) of claim 10, wherein the two sealing elements (150) seal the atomizer (100) to prevent cigarette liquid in the liquid storage chamber (160) from spilling from the liquid storage chamber (160).

12. The atomizer (100) of claim 11, wherein the atomizing pipe (170) defines two sealing grooves (172) at two opposite sides of the injection port (171), the two sealing elements (150) being received in the two sealing grooves (172) respectively.

13. An electronic cigarette (300) comprising the atomizer (100) of any one of claims 1-12.

## Patentansprüche

1. Zerstäuber (100), umfassend ein Zerstäubungsrohr (170) und eine Flüssigkeitsspeicherkammer (160), wobei der Zerstäuber (100) durch Folgendes gekennzeichnet ist:
das Zerstäubungsrohr (170) definiert eine Einspritzöffnung (171);
die Flüssigkeitsspeicherkammer (160) ist in dem Zerstäubungsrohr (170) positioniert; und
der Zerstäuber (100) umfasst ferner einen Flüssigkeitshalter (130), wobei der Flüssigkeitshalter (130) ein Einspritzfenster (131) definiert, das mit der Einspritzöffnung (171) in Verbindung stehen kann, wobei der Flüssigkeitshalter (130) auf dem Zerstäubungsrohr (170) beweglich angeordnet ist, wobei der Flüssigkeitshalter (130) senkrecht oder seitlich beweglich ist, oder in einem Kreis um das Zerstäubungsrohr (170) herum beweglich ist, oder um einen Punkt herum drehbar ist, und der Flüssigkeitshalter (130) konfiguriert ist, um einen Relativzustand der Einspritzöffnung (171) hinsichtlich einer externen Umgebung einzustellen, wobei der Relativzustand Folgendes ist: die Einspritzöffnung (171) steht mit der externen Umgebung in Verbindung oder die Einspritzöffnung (171) steht nicht mit der externen Umgebung in Verbindung.

2. Zerstäuber (100) nach Anspruch 1, wobei der Flüssigkeitshalter (130) ein Flüssigkeitshaltering ist, der auf dem Zerstäubungsrohr (170) angeordnet ist.

3. Zerstäuber (100) nach Anspruch 2, wobei das Zerstäubungsrohr (170) eine Führungsnut für den Flüssigkeitshalter (130) definiert, um sich senkrecht oder seitlich zu bewegen, und der Flüssigkeitshalter (130) in der Führungsnut verschiebbar montiert ist.

4. Zerstäuber (100) nach Anspruch 2, wobei der Flüssigkeitshalter (130) ein mit einem elastischen Element bereitgestelltes Ende aufweist; wenn der Flüssigkeitshalter (130) in Richtung des durch das elastische Element bereitgestellten Endes bewegt wird und sich die Einspritzöffnung (171) des Zerstäubungsrohrs (170) und das Einspritzfenster (131) auf dem Flüssigkeitshalter (130) überlagern, die Einspritzung von Zigarettenflüssigkeit ermöglicht wird.

5. Zerstäuber (100) nach Anspruch 1, wobei der Flüssigkeitshalter (130) ein Flüssigkeitshalteblock ist, der auf dem Zerstäubungsrohr (170) montiert ist.

6. Zerstäuber (100) nach Anspruch 5, wobei das eine Ende des Flüssigkeitshalters (130) auf dem Zerstäubungsrohr (170) durch Fixieren einer Drehwelle drehbar montiert ist, und das andere Ende des Flüssigkeitshalters (130) auf dem Zerstäubungsrohr (170) lösbar verriegelt ist.

7. Zerstäuber (100) nach Anspruch 5, wobei das Zerstäubungsrohr (170) eine Führungsnut für einen Flüssigkeitshalter (130) definiert, um sich senkrecht oder seitlich zu bewegen, und der Flüssigkeitshalter (130) in der Führungsnut verschiebbar montiert ist.

8. Zerstäuber (100) nach Anspruch 1, wobei der Zerstäuber (100) ferner wenigstens einen Anschlag (132) umfasst, der konfiguriert ist, um den Flüssigkeitshalter (130) auf dem Zerstäubungsrohr (170) lösbar zu klammern.

9. Zerstäuber (100) nach Anspruch 8, wobei der Anschlag (132) mit einer Innenwand des Flüssigkeitshalters (130) gekoppelt ist, wobei eine Außenwand des Zerstäubungsrohrs (170) eine Begrenzungsnut (177) definiert, die konfiguriert ist, um mit dem Anschlag (132) zu koppeln.

10. Zerstäuber (100) nach Anspruch 1, ferner umfassend zwei Dichtungselemente (150), die sich zwischen dem Flüssigkeitshalter (130) und dem Zerstäubungsrohr (170) befinden.

11. Zerstäuber (100) nach Anspruch 10, wobei die beiden Dichtungselemente (150) den Zerstäuber (100) abdichten, um ein Verschütten der Zigarettenflüssigkeit in der Flüssigkeitsspeicherkammer (160) aus der Flüssigkeitsspeicherkammer (160) zu verhindern.

12. Zerstäuber (100) nach Anspruch 11, wobei das Zerstäubungsrohr (170) zwei Dichtungsnuten (172) an zwei gegenüberliegenden Seiten der Einspritzöffnung (171) definiert, wobei die zwei Dichtungselemente (150) in den zwei jeweiligen Dichtnuten (172) aufgenommen werden.

13. Elektronische Zigarette (300), umfassend den Zerstäuber (100) nach einem der Ansprüche 1-12.

## Revendications

1. Atomiseur (100), comprenant un tuyau d'atomisation (170) et une chambre de stockage de liquide (160), l'atomiseur (100) étant **caractérisé par** :
le tuyau d'atomisation (170) définissant un orifice d'injection (171) ;
la chambre de stockage de liquide (160) étant positionnée dans le tuyau d'atomisation (170) ; et
l'atomiseur (100) comprenant en outre un dispositif de retenue de liquide (130), le dispositif de retenue de liquide (130) définissant une fenêtre d'injection (131) capable d'être en communication avec l'orifice d'injection (171), le dispositif de retenue de liquide (130) étant disposé de façon mobile sur le tuyau d'atomisation (170), le dispositif de retenue de liquide (130) étant mobile verticalement ou latéralement, ou mobile en cercle autour du tuyau d'atomisation (170), ou rotatif autour d'un point, et le dispositif de retenue de liquide (130) étant conçu pour régler un état relatif de l'orifice d'injection (171) par rapport à l'environnement externe, l'état relatif étant l'orifice d'injection (171) en communication avec l'environnement externe ou l'orifice d'injection (171) n'étant pas en communication avec l'environnement externe.

2. Atomiseur (100) selon la revendication 1, dans lequel le dispositif de retenue de liquide (130) est un anneau de retenue de liquide disposé sur le tuyau d'atomisation (170).

3. Atomiseur (100) selon la revendication 2, dans lequel le tuyau d'atomisation (170) définit une rainure de guidage pour que le dispositif de retenue de liquide (130) se déplace verticalement ou latéralement, et le dispositif de retenue de liquide (130) est monté coulissant dans la rainure de guidage.

4. Atomiseur (100) selon la revendication 2, dans lequel le dispositif de retenue de liquide (130) a une extrémité pourvue d'un élément élastique ;
lorsque le dispositif de retenue de liquide (130) est déplacé vers l'extrémité fournie par l'élément élastique, et que l'orifice d'injection (171) du tuyau d'atomisation (170) et la fenêtre d'injection (131) sur le dispositif de retenue de liquide (130) sont superposés, l'injection de liquide de cigarette est permise.

5. Atomiseur (100) selon la revendication 1, dans lequel le dispositif de retenue de liquide (130) est un bloc de retenue de liquide monté sur le tuyau d'atomisation (170).

6. Atomiseur (100) selon la revendication 5, dans lequel une extrémité du dispositif de retenue de liquide (130) est montée rotative sur le tuyau d'atomisation (170) par fixation d'un arbre rotatif, et l'autre extrémité du dispositif de retenue de liquide (130) est verrouillée de manière amovible sur le tuyau d'atomisation (170).

7. Atomiseur (100) selon la revendication 5, dans lequel le tuyau d'atomisation (170) définit une rainure de guidage pour que le dispositif de retenue de liquide (130) se déplace verticalement ou latéralement, et le dispositif de retenue de liquide (130) est monté coulissant dans la rainure de guidage.

8. Atomiseur (100) selon la revendication 1, dans lequel l'atomiseur (100) comprend en outre au moins un bouchon (132) conçu pour serrer de manière amovible le dispositif de retenue de liquide (130) sur le tuyau d'atomisation (170).

9. Atomiseur (100) selon la revendication 8, dans lequel le bouchon (132) est accouplé à une paroi interne du dispositif de retenue de liquide (130), une paroi externe du tuyau d'atomisation (170) définissant une rainure de limitation (177) conçue pour s'accoupler avec le bouchon (132).

10. Atomiseur (100) selon la revendication 1, comprenant en outre deux éléments d'étanchéité (150) situés entre le dispositif de retenue de liquide (130) et le tuyau d'atomisation (170).

11. Atomiseur (100) selon la revendication 10, dans lequel les deux éléments d'étanchéité (150) scellent l'atomiseur (100) pour empêcher le liquide de cigarette dans la chambre de stockage de liquide (160) de se répandre depuis la chambre de stockage de liquide (160).

12. Atomiseur (100) selon la revendication 11, dans lequel le tuyau d'atomisation (170) définit deux rainures d'étanchéité (172) sur deux côtés opposés de l'orifice d'injection (171), les deux éléments d'étanchéité (150) étant reçus dans les deux rainures d'étanchéité (172) respectivement.

13. Cigarette électronique (300) comprenant l'atomiseur (100) selon l'une quelconque des revendications 1 à 12.
